# DEMANDE DE BREVET EUROPEEN

(11) **EP 0 635 489 A1**
(43) Date de publication de la demande: **25.01.1995**
(21) Numéro de dépôt: 94401617.9
(22) Date de dépôt: 12.07.1994
(51) Int. Cl.: C07D 207/08, C07D 333/16, C08G 61/12, C25B 3/10, C25D 9/06, C02F 1/28, C02F 1/62

(54) **Dérivés de polyethers et d'hétérocycles à cinq chaînons, leur polymères et leurs applications, notamment à la complexation d'ions métalliques**

(30) Priorité: 16.07.1993 FR 9308789
(71) Demandeur: ELECTRICITE DE FRANCE Service National, F-75008 Paris (FR)
(72) Inventeur: Leclerc, Olivier, F-77250 Moret sur Loing (FR)
(74) Mandataire: Gutmann, Ernest

(57) **Abrégé**

Il s'agit d'un monomère, de formules I-III d'un polymère obtenu à partir du monomère et d'un procédé d'obtention dudit polymère. Le monomère consiste en un polyéther comportant au moins 3 motifs éther et des motifs pyrrole ou thiophène liés aux extrémités des chaînes de ce polyéther, par l'intermédiaire pour chacun de ces composés hétérocycliques, soit de leurs atomes de carbone dans les positions 3,3' de leurs hétérocycles, soit, en ce qui concerne plus particulièrement les groupes pyrrole, par l'intermédiaire de leurs atomes d'azote respectifs, les positions 2 et 2' de ces hétérocycles étant cependant libres de toutes substitutions ou tout au plus substitués par des groupes facilement éliminables (groupes de protection par exemple).

Application à l'épuration et la récupération d'ions métalliques tels que l'argent radioactif.

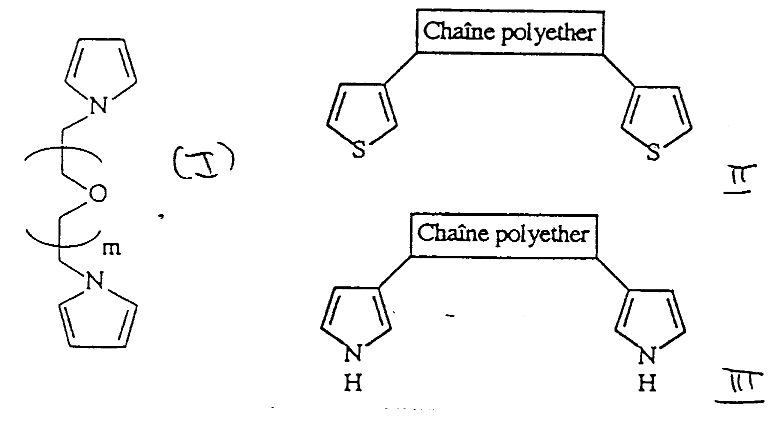

## Description

Des sels de polymères de composés à caractère aromatique possédant des cavités complexantes ont déjà été réalisés par des techniques d'électrochimie moléculaire (1), ces techniques impliquant une électrodéposition de certains polytriphénylènes sur des supports essentiellement non corrodables, tels les métaux précieux (platine, or) ou les carbones vitrifiés. L'exemple le plus courant concerne la polymérisation du composé connu sous la désignation DB-18-C-6 (I) en polytriphènylène :

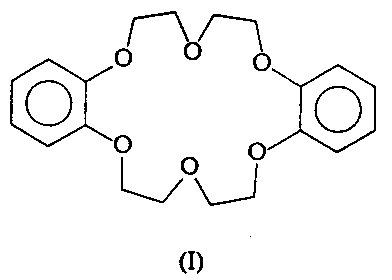

La formation directe d'une forme dopée du polymère (sans doute de nature rédox) a été démontrée. Ces sels de polymère peuvent être facilement réduits ou "dédopés" par des agents chimiques tels que les amines tertiaires, les hydroxydes de tétraalkylammonium. On peut de cette façon obtenir des résines neutres non conductrices, résistantes aux agents chimiques et aux solvants, présentant une conductivité électronique et possédant à la fois des enchaînements polyaromatiques et des motifs polyéthers, obtenus à partir de certains polyéthers bi-aromatiques (II). 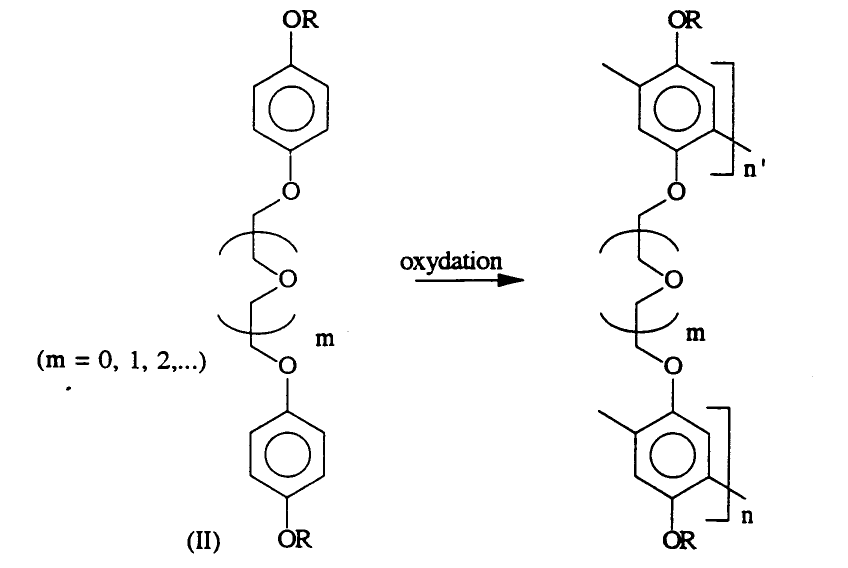
pour lesquels l'activation anodique est accrue par la présence de groupements donneurs sur les systèmes aromatiques (exemple : groupements éthers). Lorsque la polymérisation est conduite à potentiel suffisamment oxydant, des polyparaphénylènes conducteurs peuvent être obtenus grâce à l'importante concentration des sites rédox produits au sein de la matrice (oxydation à 4 électrons par motif monomère). Ces sites rédox rendent le matériau suffisamment perméable aux flux d'électrons nécessaires à la croissance en épaisseur des films formés, grâce aux sauts d'électrons induits entre ces sites, lors de l'élaboration des dépôts. Il a déjà été proposé d'utiliser de tels polymères pour extraire certains ions métalliques présents dans des solutions aqueuses ou organiques amenées à leur contact (2). Ils ne sont cependant pas toujours efficaces, s'agissant par exemple d'éliminer (ou de récupérer) des traces d'argent contenu dans certains effluents. Leurs coûts de production sont élevés. Des difficultés accompagnent aussi la synthèse des monomères, notamment à partir d'hydroquinones, puis l'oxydation de ces monomères qui doit s'effectuer à potentiel assez fortement oxydant, même si les groupements aryle ont été activés au préalable.

L'invention a pour but la production de nouveaux monomères plus aisément accessibles que les polyéthers aromatiques du type sus-indiqué, polymérisables par la mise en oeuvre de techniques d'électrochimie moléculaire du genre susdit, mais à des potentiels moins oxydants. L'invention a par conséquent également pour but l'extension aisée de ces techniques à d'autres catégories de supports conducteurs plus sensibles à l'oxydation que les métaux précieux sus-indiqués. En particulier, l'invention a pour but la formation plus aisée qu'auparavant de dépôts de ce type sur des supports de graphite. L'utilisation de celui-ci comme support pour les polyéthers aromatiques de type II était difficile, en raison de la capacité du graphite à se transformer au moins partiellement en sels de graphite à des potentiels d'oxydation relativement faibles, parfois inférieurs aux potentiels requis pour l'obtention d'une électrodéposition des polymères correspondants.

L'invention a encore pour but la production de dérivés de polyéthers particulièrement performants, pour ce qui est de leur capacité à éliminer des traces de certains métaux, en concentrations inférieures à 10⁻¹⁰M, voire même 10⁻¹²M. En particulier les métaux dont il est question sont ceux dont la séquestration ou la cryptation est recherchée : notamment les métaux de transition, zinc, cadmium, argent, ... ou éléments métalliques radioactifs.

Les monomères selon l'invention, également polymérisables par des techniques d'électrochimie moléculaires, consistent en des polyéthers comportant au moins trois motifs ether, porteurs de motifs pyrrole ou thiophène liés aux extrémités des chaînes de ces polyéthers, par l'intermédiaire pour chacun de ces composés hétérocycliques, soit de leurs atomes de carbone dans les positions 3,3' de leurs hétérocycles, soit, en ce qui concerne plus particulièrement les groupes pyrrole, par l'intermédiaire de leurs atomes d'azote respectifs, les positions 2 et 2' de ces hétérocycles étant cependant libres de toutes substitutions ou tout au plus substitués par des groupes facilement éliminables (groupes de protection par exemple).

Les chaînes polyéthers entrant dans la constitution des monomères selon l'invention sont de préférence linéaires et contiennent des motifs éther de préférence du type -[(CH₂)ₚ-O-]ₙ- avec p étant un nombre entier de 2 ou 3 et n étant un nombre entier de 3 à 5, ces chaînes étant alors reliées en α et en ω à des motifs pyrrole ou thiophène dans les conditions sus-indiquées.

En particulier, ces chaînes sont du type :

-(CH₂)_{q'} - [ (CH₂)_{p'} - O - (CH₂)_{p''}]ₘ - (CH₂)_{q''}-

avec
- q' et q'' étant, indépendamment l'un de l'autre, égaux à 1 ou 2,
- p et p'' étant, indépendamment l'un de l'autre, égaux à 1 ou 2.

L'invention concerne plus particulièrement de nouveaux monomères de formule :

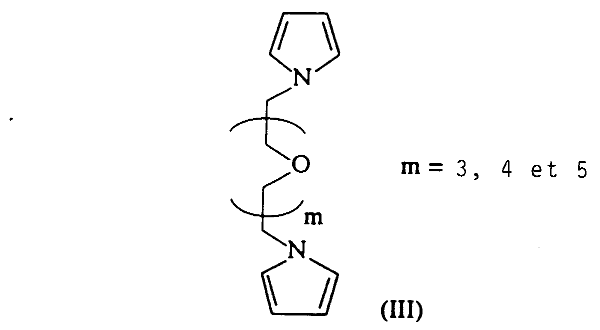

De tels monomères sont susceptibles d'être obtenus facilement par réaction d'un sel de pyrrolyl-métal alcalin, notamment pyrrolyl-potassium sur un dérivé dihalogéné du glycol correspondant à la chaîne de polyéther finale. Par exemple, le glycol correspondant aura préalablement été transformé en dérivé dichloré par réaction avec le chlorure de thionyle.

D'autres catégories de monomères préférés conformes à l'invention sont caractérisées par l'une ou l'autre des structures suivantes : 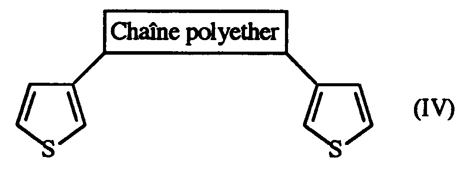 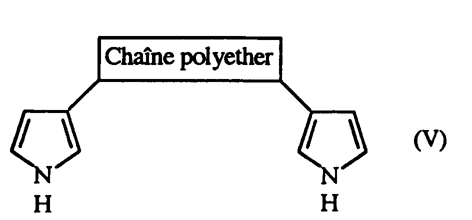
et plus particulièrement ceux répondant à la formule suivante : 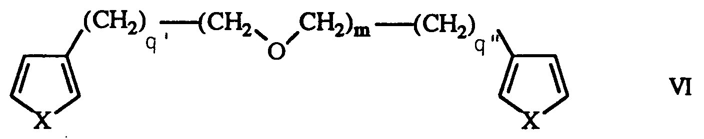
avec X étant un groupe NH ou un atome d'azote ou de soufre, q' et q'' étant, indépendamment de l'autre, égaux à 1 ou 2 et m étant égal à un nombre entier de 3 à 5.

L'intérêt de telles molécules dans le processus de polymérisation anodique est de donner lieu à des rendements de polymérisation hétérogène [(rapport du poids de résine électrodéposée/masse de monomère) x 100] beaucoup plus importants. Il convient de laisser les positions en 2,2' sur les noyaux pyrrole et thiophène, libres de façon à ne pas ralentir le processus de polymérisation.

Ainsi, avec les thiophènes et lorsque p=0 et p'=0, l'accrochage d'une chaîne polyéther peut se faire sans problème à partir du 3-bromothiophène conformément au modèle de réaction développé ci-dessous.

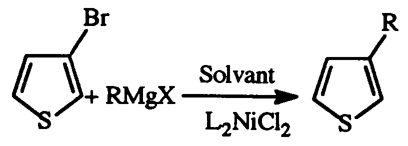

Cette réaction est conduite à la température ordinaire dans l'éther ou le THF pendant 24 heures. X (dans l'halogènure de départ) RX(= Cl ou Br). L est un ligand de type phosphine (Réf. : M. KUMADA et al., J. Org. Chem., (1981), 46, 4481) et J.P. MONTHEARD et al., Synth. Comm., (1984), 14, 289). En l'occurrence, le dérivé organomagnésien formé sera un dérivé "di-organomagnésien" du type R'(MgX)₂ qui peut être facilement réalisé à partir de α,ω-diols comportant une chaîne polyéther, dichlorés par le chlorure de thionyle.

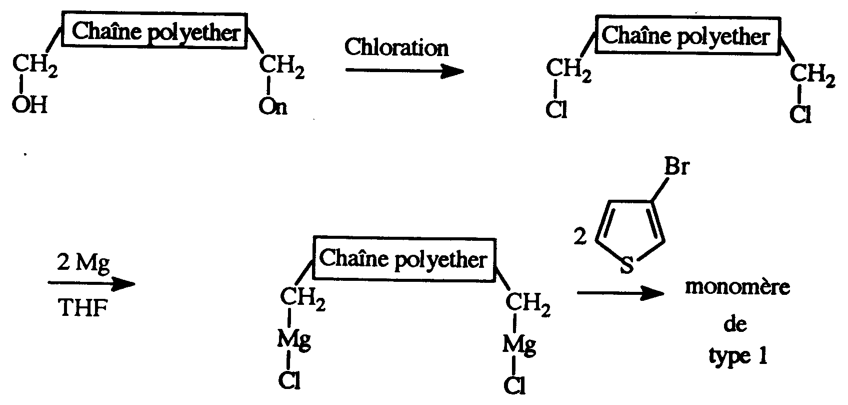

La réaction de substitution en position 3 des pyrroles (en particulier dans le cas où p=0 et p'=0) est en principe plus délicate car elle suppose a priori, la protection de la fonction N-H-. Un mode opératoire possible est la protection par un chlorure de sulfonyle (R = méthyle ou phényle) : 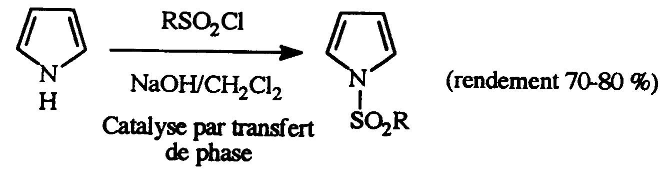
puis de réaliser une réaction de Friedel et Crafts sur le chlorure d'acétyle en présence de chlorure d'aluminium. La cétone est ensuite réduite en alcool correspondant avec un bon rendement par l'hydrate de lithium et d'aluminium.

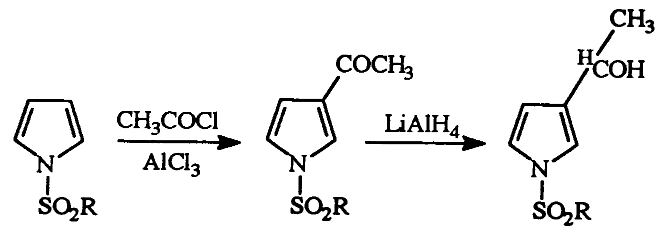

La réaction d'éthérification est conduite dans le THF en présence d'hydrure de sodium alors que le ditosylate du diol polyéther est ajouté progressivement. La déprotection de la fonction aminée du pyrrole est alors réalisée de façon classique par catalyse par transfert de phase (présence d'une base en milieu biphasique en présence d'un détersif, tel que celui commercialisé sous la marque TRITON B.

L'introduction de groupements acides (carboxylique et sulfonique en position 3') sur le pyrrole et le thiophène permet de renforcer considérablement le caractère de résine échangeuse d'ion de ce type de polymère.

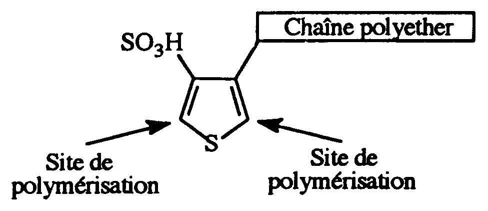

Cependant, la difficulté relative de la synthèse de tels monomères exclut a priori leur utilisation. Il est préféré de réaliser une coopolymérisation de : 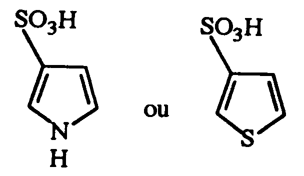
avec les monomères polyéthers 1 ou 2 en mélanges équimoléculaires (il y a ainsi introduction statistique de 0,5 groupement sulfonique par cycle aromatique).

Dans le cas des composés de formule VI dans lesquels p et p' sont différents de zéro, les composés thiophène ou pyrrole correspondants initiaux présenteront avantageusement des groupes OH en 2 et 2' respectivement, lesquels sont alors aptes à intervenir dans une réaction avec un ditosylate polyéther correspondant à la chaîne polyéther devant lier ces hétérocycles entre eux.

L'invention concerne aussi les oligomères (dimères inclus) et les polymères susceptibles d'être produits à partir des susdits monomères, ces polymères étant caractérisés par des chaînes polymériques contenant une pluralité de cycles pyrrole ou thiophène, le cas échéant les deux à la fois et interconnectés entre eux via des liaisons 2,2', d'une part, et des chaînes de polyéthers, répondant en particulier aux formules -CH₂-(-CH₂-O-CH₂-)ₘ-CH₂- avec m égal à 3, 4 ou 5, ces chaînes étant elles-mêmes liées par leurs propres extrémités, soit aux atomes de carbone en positions respectivement 3,3' de ces cycles pyrrole ou thiophène, soit, dans le cas des cycles pyrrole, à leurs atomes d'azote.

Un procédé de production de ces polymères comprend l'électrodéposition du polymère sur une anode d'une cellule d'électrolyse, ou sur un matériau de support conducteur, notamment à base de carbone en contact électrique avec cette anode, à partir d'un électrolyte contenant le monomère tel que défini plus haut et d'un sel susceptible de donner naissance à un anion non oxydable, sous un potentiel autorisant une polymérisation du monomère au contact de l'anode ou, lorsqu'il est présent, du matériau de support, les susdits monomères et sels étant à l'état dissous dans un solvant organique polaire ou dipolaire, ce solvant et le support étant respectivement choisis parmi ceux qui présentent eux-mêmes une résistance suffisante à l'oxydation sous le susdit potentiel.

Il va sans dire que ce procédé doit être mis en oeuvre en l'absence de toute trace du métal ou des métaux particuliers, notamment métaux alcalins ou alcalino-terreux, métaux de transition, zinc, cadmium, argent, ..., ou éléments radioactifs, lorsque les polymères produits sont destinés à être mis en oeuvre pour la séquestration ou cryptation de ces métaux, notamment sous forme d'ions métalliques, plus particulièrement à l'état de traces, dans des milieux liquides amenés à leur contact, à des fins soit d'épuration, soit de récupération.

La polymérisation des monomères dipyrrole ou dithiophène peut être réalisée sur toute surface non corrodable telle que le platine, l'or, le rhodium. Le dépôt est également possible sur tous carbones vitreux et différents types de graphite [naturel ou "HOPG" (abréviation de l'expression anglaise "Highly oriented pyrolytic graphite" ; graphite pyrolytique fortement orienté)]. Elle peut être conduite facilement dans les solvants organiques polaires et dipolaires usuels, s'ils présentent une activité électrochimique réduite (par l'oxydation importante du solvant avant +1,2V/Ag/Ag⁺). Ainsi, l'acétonitrile technique peut être facilement utilisé, mais le dichlorométhane et le nitrométhane peuvent conduire aussi à la polymérisation des pyrroles. L'électrolyte doit essentiellement comporter un anion non oxydable. Ainsi, les perchlorates, hexafluorophosphates, tétrafluoroborates, hexafluoro- acétates, etc..., de tétraalkylamonium (alkyl = Me, Et, Pr, Bu, Hex, d, etc...) et de métaux alcalins (sels de lithium, sodium, potassium) sont tous des électrolytes qui peuvent être utilisés. Le choix du sel peut être guidé par la solubilité dans le solvant considéré. Les polymères se déposent particulièrement bien. Ils peuvent être obtenus sur les différentes formes de carbone, en particulier de graphite, et peuvent être obtenus sous forme de dépôts beaucoup plus épais que sur les métaux précieux.

L'invention concerne évidemment aussi les polymères supportés par ces supports conducteurs, notamment à base de carbone et plus particulièrement de graphite. L'utilisation de supports de carbone, en particulier de graphite est d'un intérêt particulier compte-tenu de la capacité de ces matériaux à être amenés sous des formes diverses, notamment en grains poreux qui peuvent alors être chargés en polymères.

L'invention concerne naturellement aussi les applications qui peuvent être faites de ces polymères à l'état supporté ou non. En particulier, l'invention concerne l'application de ces polymères en tant qu'agents de séquestration ou "cryptation" d'ions, métalliques, plus particulièrement issus de métaux de transition, de zinc, de cadmium, d'argent, etc... contenus dans des solutions amenées à leur contact, à des fins soit, d'épuration de ces solutions, soit même de récupération des métaux correspondants. Les polymères du genre en question peuvent alors être considérés comme des "polymères-cages" vis à vis de ces ions.

Les oligomères de faibles poids moléculaires, et plus particulièrement les dimères, notamment dimères cycliques assez solubles dans des solvants organiques, peuvent de ce fait être l'objet d'applications industrielles diverses : catalyse en transfert de phase, application à la séparation des terres rares, etc...

D'autres caractéristiques de l'invention apparaîtront encore au cours de la description qui suit de certains exemples qui n'ont d'autre objectif que celui d'illustrer l'invention, sans pour autant la limiter. Il sera aussi fait référence aux dessins dans lesquels :
- la **figure 1** présente des spectres de résonance magnétique nucléaire relatifs à un monomère particulier conforme à l'invention :
   spectres de RMN ¹³C 75MHz(CDCl₃)δ(ppm/TMS) ;
- la **figure 2** est un schéma d'une cellule électrolytique susceptible d'être mise en oeuvre pour la production d'un polymère conforme à l'invention, notamment à l'état supporté sur un support conducteur ;
- la **figure 3** est un schéma illustrant le suivi de la formation de la couche de polymère dans la cellule d'électrolyse en cours de fonctionnement.
- les **figures 4 et 5** sont des reproductions de photographies faisant apparaître la morphologie de dépôts de polymère obtenus sur grains de graphite dans les conditions décrites dans l'exemple, après dédopage par Bu₃N contenu dans CH₃CN, à des grossissements différents, respectivement 18700 et 16000 fois. Les échantillons ont été dorés à l'or fin.
- la **figure 6** montre un schéma d'un mode de réalisation de filtre constitué en mettant en oeuvre le procédé de production d'un polymère selon l'invention.

### EXEMPLES:

### A) Préparation des monomères (III)

Le mode de synthèse applicable à l'ensemble des polyéthers (1 ≦ m ≦ 5) est décrit ci-après.

### 1. Exemple de synthèse type pour le triéther (m=3):1-11bis(1-1'pyrrole) 3,6,9 trioxaundecane

Il convient de synthétiser tout d'abord le pyrrole potassium :

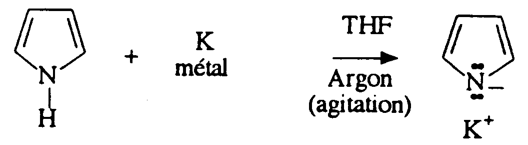

Dans un ballon de 250 ml (3 tubulures), on place 40 ml de THF préalablement traité par le système benzophénone-sodium pour éliminer les péroxydes. On ajoute, par petite portion, 8 g de potassium (2,05 10⁻¹ mole). Ensuite par l'ampoule à brome, on ajoute goutte à goutte 14,42 g de pyrrole (2,15 10⁻¹ mole). On laisse sous l'agitation (à la température ambiante) dans une atmosphère inerte (argon) pendant 24 heures. Le contenu est filtré, lavé à l'éther pus séché. De cette façon 16,5 g de sel sec sont obtenus (rendement 75 % environ).

Parallèlement, le biélectrophile est obtenu par traitement de tétraéthylène glycol par SOCl₂ selon le mode opératoire décrit par C.J. Pedersen (J. Am. Chem. Soc. (1967), 89, 7017):

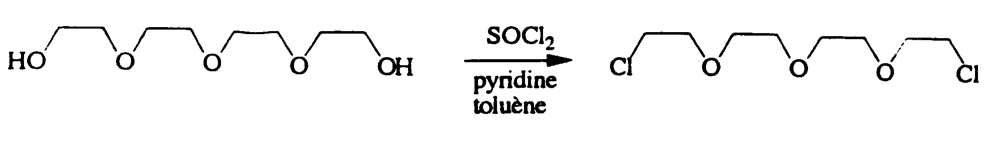

La réaction se fait en ballon de 500 ml (avec réfrigérant, arrivée d'argon, thermomètre et ampoule à brome) dans lequel on place 43 g de tétraéthylène glycol (0,22 mole), 200 ml de toluène et 38,6 g de pyridine (0,49 mole). Le mélange est porté, sous agitation, à 85°C et on ajoute très lentement 58 g de chlorure de thionyle (0,49 mole). Cela nécessite environ 4 heures ; par la suite la température du milieu réactionnel est maintenue à 80°C pendant 20 heures. On ajoute ensuite une solution de HCl (5 ml d'acide concentré dans 20 ml d'eau). On décante, lave à l'eau, sèche sur CaCl₂. De cette façon, 46 g de liquide brut sont obtenus. Par distillation sous vide, 40 g de produit dichloré pur sont isolés (rendement 80 %) . Ensuite, la condensation du sel de pyrrole sur le biélectrophile est réalisée pour conduire au monomère.

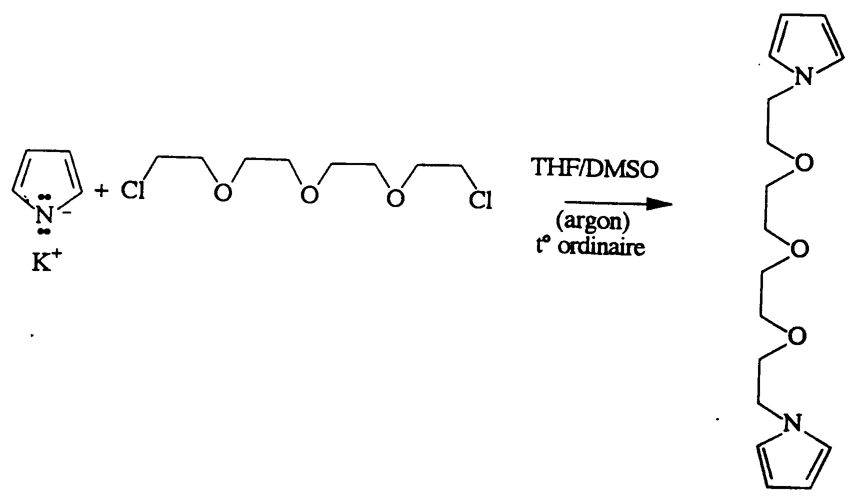

La condensation est réalisée dans un ballon de 250 ml à 3 tubulures dans lequel on dispose :
- 25 ml de diméthylsulfoxyde (DMSO),
- 15,75 g de pyrrole K(1,5 10⁻¹ mole) donc en excès de 50 %
- 50 ml de THF traité et distillé.

Le biélectrophile chloré 11,55 g (0,5 10⁻¹ mole) en solution dans 40 ml de THF est ajouté goutte à goutte sous argon. On laisse sous agitation à température ordinaire pendant 20 heures. Ensuite, on évapore le THF, on reprend à l'éther, lave à l'eau, décante, sèche, évapore. 16 g de liquide huileux sont alors distillés sous vide permettant l'obtention de 11 g de monomère (III) (Rendement : 75 % par rapport au dérivé dichloré).

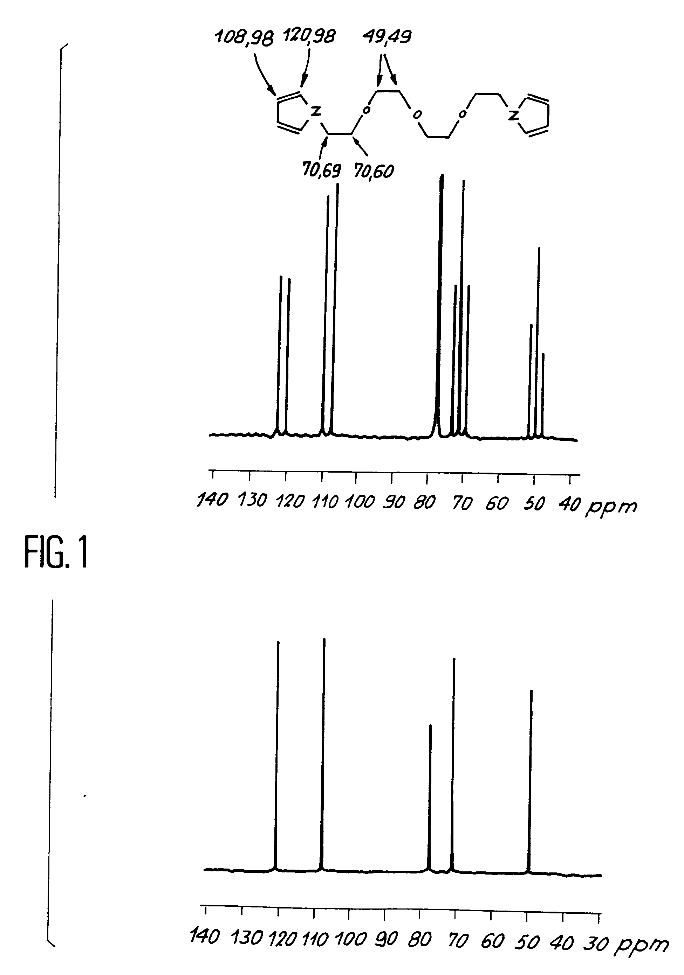

Des spectres de RMN du produit obtenu sont montrés à la **figure 1**.

### B) Conditions propres à la polymérisation électrochimique des monomères (III)

Deux modes de polymérisation sont décrits ci-après :

### 1. Electrolyse potentiostatique sur feuille de platine

1,2 g de monomère (III) (m=3) est dissout dans 25 ml de chlorure de méthylène contenant Bu₄NBF₄ 0.1 M. Cette solution constitue l'anolyte disposé dans un compartiment d'une cellule en U comportant un deuxième compartiment (volume 20 ml). Les deux compartiments sont séparés par un fritté de porosité 3. L'anode est constituée d'une feuille de platine de surface totale de 20 cm². La cathode est en graphite (surface immergée 15 cm²). Un potentiel contrôlé de +1.0 V est appliqué à l'anode. Un courant de 150 mA passe pendant 3h30, 180 mg d'un dépôt conducteur noir brillant sont obtenus.

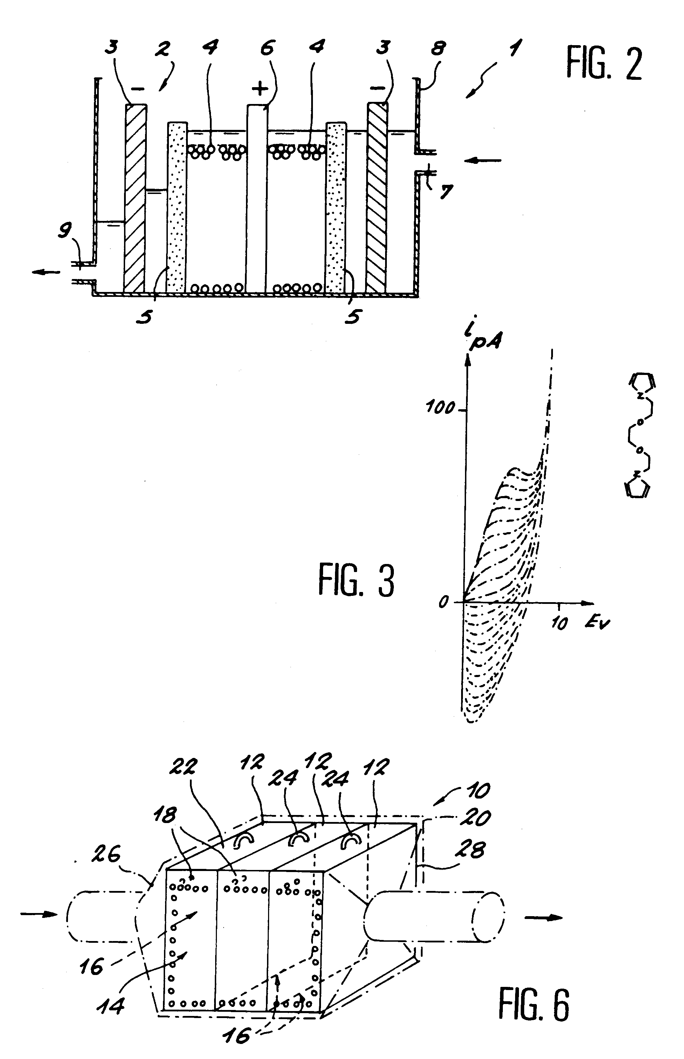

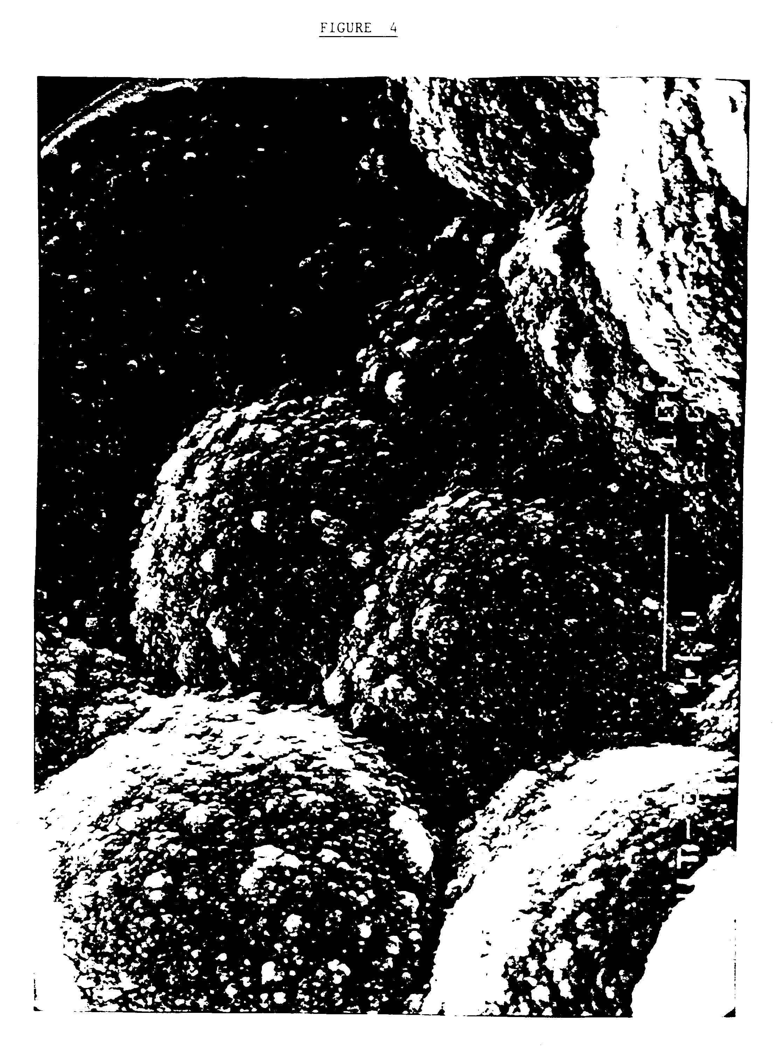

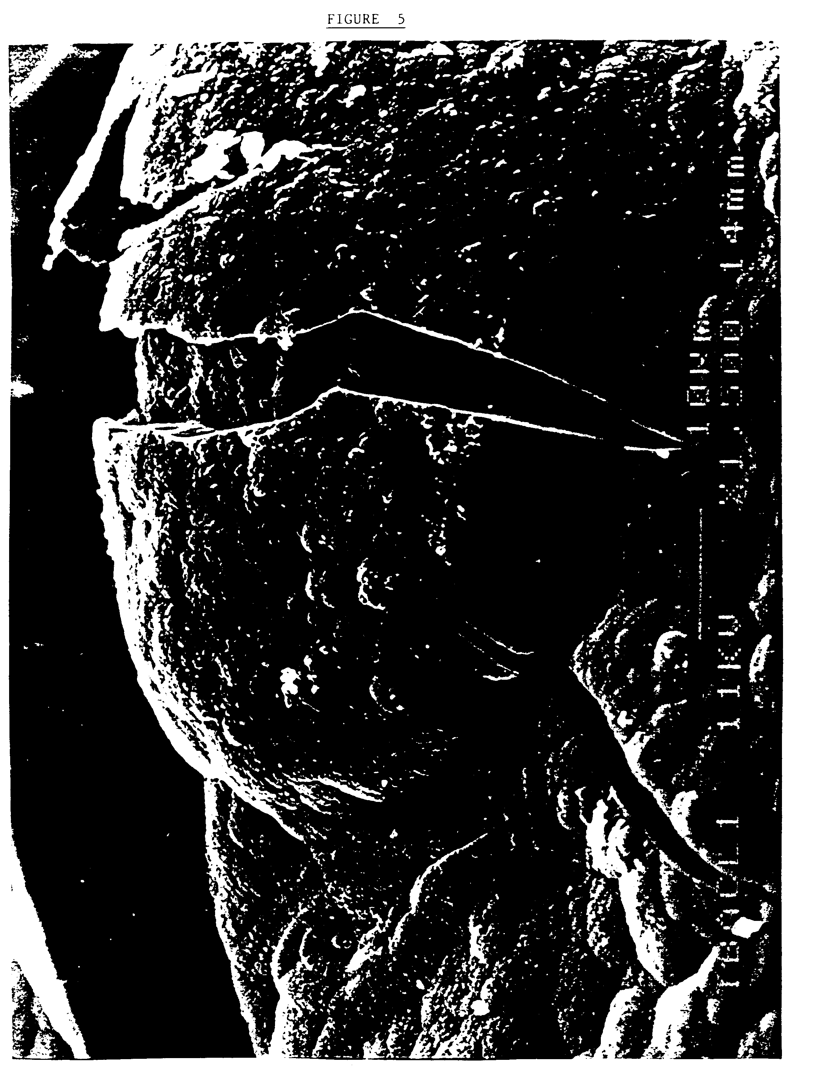

La formation d'un polymère conducteur à partir de III (m=2) peut être démontrée par voltamétrie cyclique (**figure 3**), et ce dans les conditions suivantes : concentration 5.10⁻³mole 1⁻¹. Electrolyte : acétonitrile contenant Bu₄NBF₄ 0.1 mole.1⁻¹. Microanode de platine polie (surface : 0,87 mm²). Vitesse de balayage : 0.5 V.s⁻¹. Potentiels indiqués par référence au système Ag/AgNO₃ 0.1 M. Evolution du dépôt au cours des 27 premiers balayages (on notera la progression régulière du film dont la réponse rédox se situe à +0.5 V.

### 2. Electrolyse intensiostatique sur billes ou grains de graphite

Une solution de 1,7.10⁻² mol/l de (III) (m=3) dans l'acétonitrile contenant Bu₄NBF₄ 0,1 M est disposée dans une cellule d'électrolyse de type PRIAM 1. La circulation de l'électrolyte est maintenue constante pendant l'opération de polymérisation à un débit de 2 l/min.

On a représenté schématiquement sur la figure 2 une cellule 1 d'électrolyse de ce type.

La cathode 2 est composée soit d'une ou de plusieurs grille(s) 3 d'inox (surface apparente : 1 dm²) ou encore d'un papier de carbone (par exemple le produit papier de Carbone Lorraine). Le coke de pétrole 4 (masse totale : 50 g) est disposé au centre de la cassette pour former une anode volumique entre deux parois poreuses 5, servant d'espaceur. L'amenée de courant anodique est une grille 6 de titane recouverte d'oxyde d'irridium.

L'électrolyte 7 entre en partie supérieure de la cuve de rétention 8 et sort en partie inférieure 9 de l'autre côté.

L'intensité imposée est de 0,6 A pendant 6 heures. Dans ce cas, le dépôt de polymère obtenu est de 3,5 g. Il est caractérisé par RPE, IR et MEB (**figures 4 et 5**).

Les valeurs données ci-dessus le sont à titre d'exemple nullement limitatif.

Par rapport à une polymérisation par électrolyse potentiostatique une polymérisation par électrolyse intensiostatique est représentative d'une préparation en milieu industriel. De plus, comme on le verra par la suite, la technique d'électrolyse mise en oeuvre permet de préparer directement des éléments qui seront ensuite assemblés pour faire des modules de récupération de cations métalliques par cryptation avec les résines polymères.

### 3. Réduction et dédopage des dépôts conducteurs

Comme il a été expliqué dans des documents antérieurs (3, 4), il est nécessaire d'effectuer une réduction du polymère pour éliminer une partie des anions provenant de l'électrolyte. Cette réduction peut se faire par voie chimique ou électrochimique. Il a été montré que le dédopage du polymère était plus complet et plus simple par voie chimique que par voie électrochimique. De plus, cette technique peut être appliquée à l'ensemble des polymères synthétisés.

Le dédopage des dépôts sur plaque métallique ou sur carbone peut être facilement obtenu traitement, après la fin de l'électrolyse. Lavage par l'acétonitrile et séchage à l'étuve à vide à température < 100°C. Le traitement est réalisable parles hydroxydes de tétrabutyl ou tétraéthylammonium commerciaux (provenance Fluka ou Aldrich) à la concentration 10⁻¹ou 10⁻² M dans l'acétonitrile. La réduction peut également être réalisée par les amines tertiaires ou les superoxydes d'ammonium (fait in situ par électrolyse de l'oxygène) ou d'alcalins (superoxyde de potassium).

Néanmoins, il apparaît nécessaire de faire subir, pour toute application relative à une extraction de cations métalliques, un traitement à l'hydroxyde de tétraalkylammonium qui rend plus facile l'échange entre ions ammonium et ions métalliques.

les polymères formés à partir de pyrroles éthers de type III (mettant en oeuvre la capacité du pyrrole à se polymériser en 2-2'), présentent des structures à maille possédant un nombre (probablement important) de sites polyéthers.

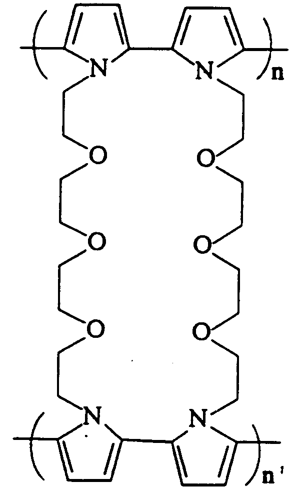

La méthode anodique permet donc la formation facile de sites à 2, 4, 6, 8 ou 10 oxygènes. Par ailleurs, des électrolyses mixtes de deux monomères peut aisément moduler la taille des sites.

### Exemple :

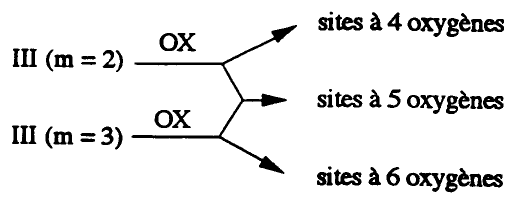

De plus, la formation de macrosites à partir non plus de 2, mais de 3 voire 4 monomères n'est plus exclue et peut contribuer à des applications dans l'extraction de cations complexes. Exemple pour (III), m=2 :

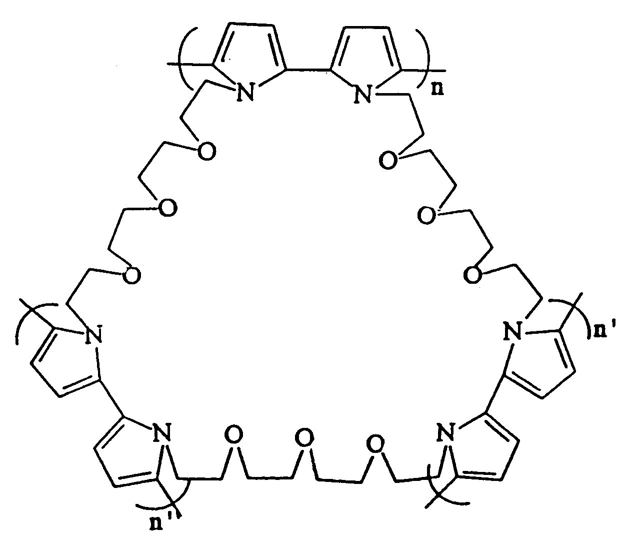

Il est également revendiqué la possibilité d'obtenir non seulement ces résines sous forme dopée, mais sous forme neutre.

Ces polymères réduits, sous forme neutre, peuvent se comporter comme des résines échangeuses d'ions, notamment présenter la faculté d'extraction d'ions métalliques présents dans les solutions aqueuses ou organiques à condition que les résines soient préalablement traitées par des sels d'ammonium (fonctionnement en résine échangeuse d'ions). Il est revendiqué l'affinité des résines pour les ions alcalins, alcalino-terreux, l'ion argent et les ions de métaux précieux.

### C) Production et utilisation industrielle des résines polymériques synthétisées

La méthode de synthèse industrielle mise en oeuvre peut être celle décrite dans le paragraphe 2. soit une électrolyse intensiostatique sur billes de graphite. En effet, l'usage d'anodes volumiques au sein desquelles circule l'électrolyte chargé en motifs monomères va permettre au cours de l'électrolyse d'obtenir des éléments poreux fortement chargés en résines polymériques "cryptantes". En fin d'électrolyse, le dédopage des motifs polymères se fait dans la même boucle réactionnelle.

Si le réacteur d'électrolyse utilisé est un réacteur à électrode volumique, il suffit après le dédopage du polymère d'ôter les anodes volumiques de la cuve d'électrolyse, de retirer l'amenée de courant puis de clore le sommet de l'assemblage en polypropylène qui contient les granules carbonés enrobés de résine polymère. Il suffit ensuite de réunir plusieurs anodes volumiques dans un réceptacle approprié pour former un empilement poreux apte à la cryptation de cations métalliques présents en faible concentration dans les effluents industriels.

L'épuisement en ions métalliques se fera par une simple percolation dans cet empilement.

La présente invention concerne également un procédé de fabrication d'une cartouche filtrante d'épuration et/ou de récupération d'ions métalliques contenus dans une solution, caractérisé en ce que
on produit par électrolyse intensiostatique des motifs polymères à partir d'un électrolyte chargé en motifs monomères du type décrit ci-dessus et notamment consistant en un polyéther porteur de motifs pyrrole ou thiophène liés aux extrémités des chaînes de ce polyéther, par l'intermédiaire pour chacun de ces composés hétérocycliques, soit de leurs atomes de carbone dans les positions 3,3' de leurs hétérocycles, soit, en ce qui concerne plus particulièrement les groupes pyrrole, par l'intermédiaire de leurs atomes d'azote respectifs, les positions 2 et 2' de ces hétérocycles étant cependant libres de toutes substitutions ou tout au plus substitués par des groupes facilement éliminables, sur un matériau support conducteur à base de carbone contenu dans un réservoir à parois poreuses appartenant à une anode volumique,
en fin d'électrolyse, on dédope les motifs polymères ainsi obtenus dans la même boucle réactionnelle,
après dédopage des motifs polymères, on retire les moyens d'amenée du courant du réservoir,
puis on obture ledit réservoir qui contient le matériau support enrobés des motifs polymères, pour obtenir ainsi ladite cartouche filtrante.

La présente invention concerne enfin un filtre d'épuration et/ou de récupération d'ions métalliques, plus particulièrement de métaux lourds tels que l'argent, contenus dans des solutions amenées à son contact, caractérisé en ce qu'il comprend au moins une cartouche filtrante comprenant un réservoir à parois poreuses contenant un matériau conducteur, notamment à base de carbone, enrobé de résine polymère du type décrit ci-dessus et notamment de résine polymère caractérisée par des chaînes polymériques contenant une pluralité de cycles pyrrole ou thiophène, le cas échéant les deux à la fois et interconnectés entre eux via des liaisons 2,2', d'une part, et des chaînes de polyéthers, répondant en particulier aux formules -CH₂-(-CH₂-O-CH₂-)ₘ-CH₂- avec m égal à 3, 4 ou 5, ces chaînes étant elles-mêmes liées par leurs propres extrémités, soit aux atomes de carbone en positions respectivement 3,3' de ces cycles pyrrole ou thiophène, soit, dans le cas des cycles pyrrole, à leurs atomes d'azote.

La **figure 6** montre schématiquement un mode de réalisation d'un tel filtre 10, selon l'invention comprenant trois cartouches filtrantes 12 identiques, par exemple de forme parallélépipédique.

Chaque cartouche comprend un ceinture latérale 14 en matériau rigide, de maintien structurel de la cartouche et deux parois rectangulaires 16 en matériau poreux, par exemple en polypropylène les cartouches contiennent un matériau conducteur 18 à base de carbone enrobé de résine polymère du type selon l'invention.

Le filtre comprend une enveloppe 20 parallélépipédique (en trait mixte sur la figure) par exemple en acier inox, contenant les trois cartouches.

Il est muni d'une plaque supérieure 22 amovible, ce qui permet l'enlèvement et le remplacement des cartouches filtrantes munie en partie supérieure d'une poignée 24 de préhension.

Le filtre comprend par ailleurs une portion par exemple tronconique 26 d'entrée du liquide à décontaminer, et une portion 28 identique de sortie du liquide après décontamination.

Un tel filtre permet par exemple d'obtenir une décontamination en continu d'effluent chargé en Ag110 radioactif avec un débit de l'ordre du m³/h.

### REFERENCES

**(1)** J. Simonet et J. Rault-Berthelot, Prog. Solid St. Chem., (1991), 21, 1-48 (et références citées).
**(2)** V. Le Berre, L. Angely, N. Simonet-Gueguen et J. Simonet, J. Electroanal. Chem., (1986), 206, 115.
**(3)** J. Simonet et C. Saboureau, Demande de brevet français (1990).
**(4)** Y. Gache et J. Simonet, J. Chim. Phys., (1992), 89, 1027.

## Revendications

1. Monomère consistant en un polyéther comportant au moins 3 notifs ether et des motifs pyrrole ou thiophène liés aux extrémités des chaînes de ce polyéther, par l'intermédiaire pour chacun de ces composés hétérocycliques, soit de leurs atomes de carbone dans les positions 3,3' de leurs hétérocycles, soit, en ce qui concerne plus particulièrement les groupes pyrrole, par l'intermédiaire de leurs atomes d'azote respectifs, les positions 2 et 2' de ces hétérocycles étant cependant libres de toutes substitutions ou tout au plus substitués par des groupes facilement éliminables (groupes de protection par exemple).

2. Monomère selon la revendication 1, caractérisé en ce que la chaîne polyéther entrant dans la constitution de ce monomère est linéaire et contient des motifs éther de préférence du type -[(CH₂)ₚ-O-]ₙ- avec p étant un nombre entier de 2 ou 3 et n étant un nombre entier de 3 à 5, cette chaîne étant reliée en α et en ω à des motifs pyrrole ou thiophène.

3. Monomère selon la revendication 2, caractérisé en ce que la chaîne est du type :
-(CH₂)_{q'} - [ (CH₂)_{p'} - O - (CH₂)_{p''}]ₘ - (CH₂)_{q''}-
avec
- q' et q'' étant, indépendamment l'un de l'autre, égaux à 1 ou 2,
- p et p'' étant, indépendamment l'un de l'autre, égaux à 1 ou 2.

4. Monomère selon l'une quelconque des revendications 1 à 3, caractérisé par la formule : 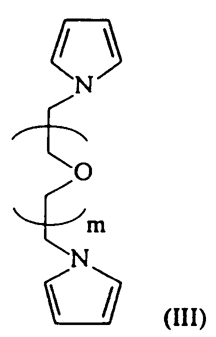 avec m = 3, 4 ou 5

5. Monomère selon l'une quelconque des revendications 1 à 3, caractérisé par l'une ou l'autre des structures suivantes :

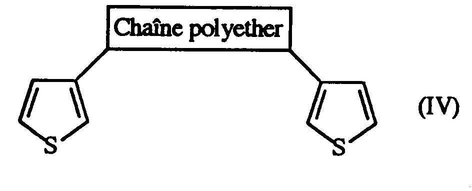

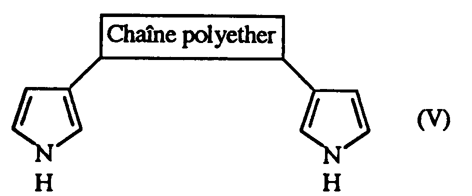

6. Monomère selon la revendication 5, caractérisé en ce qu'il répond à la formule suivante : 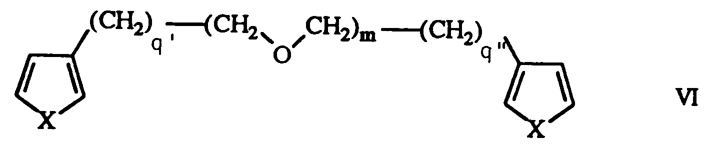 avec X étant un groupe NH ou un atome de soufre, m étant égal à un nombre entier de 3 à 5, q' et q'' étant, indépendamment l'un de l'autre, égaux à 1 ou 2.

7. Monomère selon l'une quelconque des revendications 1 à 6, caractérisé en ce qu'il porte un groupe acide, notamment carboxylique ou sulfonique, dans les positions 3' de ses groupes hétérocycliques.

8. Monomère selon l'une quelconque des revendications 1 à 7, caractérisé en ce qu'il se trouve sous forme de sel pyrrolyl-alcalin ou thiophényl-alcalin, notamment de sel de pyrrolyle ou thiophényle de potassium.

9. Polymère caractérisé par des chaînes polymériques contenant une pluralité de cycles pyrrole ou thiophène, le cas échéant les deux à la fois et interconnectés entre eux via des liaisons 2,2', d'une part, et des chaînes de polyéthers, répondant en particulier aux formules -CH₂-(-CH₂-O-CH₂-)ₘ-CH₂- avec m égal à 3, 4 ou 5, ces chaînes étant elles-mêmes liées par leurs propres extrémités, soit aux atomes de carbone en positions respectivement 3,3' de ces cycles pyrrole ou thiophène, soit, dans le cas des cycles pyrrole, à leurs atomes d'azote.

10. Polymère selon la revendication 9, caractérisé en ce qu'il est sous forme déposée sur un support conducteur, notamment à base de métal non corrodable, de carbone et plus particulièrement de toutes formes de graphite.

11. Polymère selon la revendication 9, caractérisé en ce qu'il est exempt de toute trace d'ion métallique.

12. Procédé de production d'un polymère selon la revendication 7 ou 8, caractérisé en ce qu'il comprend l'électrodéposition du polymère sur une anode d'une cellule d'électrolyse, ou sur un matériau de support conducteur, notamment à base de carbone en contact électrique avec cette anode, à partir d'un électrolyte contenant le monomère tel que défini plus haut et d'un sel comportant un anion non oxydable, à un potentiel autorisant une polymérisation du monomère au contact de l'anode ou, lorsqu'il est présent, du matériau de support, les susdits monomères et sels étant à l'état dissous dans un solvant organique polaire ou dipolaire, ce solvant et le support étant respectivement choisis parmi ceux qui présentent eux-mêmes une résistance suffisante à l'oxydation au susdit potentiel.

13. Procédé selon la revendication 12, caractérisé en ce que la réaction est opérée en l'absence de toute trace de métal sous forme ionique ou pouvant être complexée

14. Procédé de production d'un polymère selon la revendication 12 ou 13, caractérisé en ce que le support est en billes de graphite.

15. Procédé de production d'un polymère selon l'une quelconque des revendications 12 à 14, caractérisé en ce que l'électrolyte utilisée dans ladite cellule d'électrolyse comprend des sels anioniques de tétraalkylamonium.

16. Application des polymères selon l'une quelconque des revendications 9 à 11 ou du polymère obtenu par le procédé selon l'une quelconque des revendications 12 à 14, à la séquestration ou "cryptation" d'ions métalliques, plus particulièrement de métaux de transition, de zinc, de cadmium ou d'argent, contenus dans des solutions amenées à leur contact, à des fins soit d'épuration de ces solutions, soit même de récupération des métaux correspondants.

17. Application selon la revendication 16, caractérisé en ce que le polymère est totalement exempt de l'ion ou des ions métalliques dont la séquestration ou la cryptation est recherchée.

18. Procédé de fabrication d'une cartouche filtrante d'épuration et/ou de récupération d'ions métalliques contenus dans une solution, caractérisé en ce que
on produit par électrolyse intensiostatique des motifs polymères à partir d'un électrolyte chargé en motifs monomères selon l'une quelconque des revendications 1 à 8, sur un matériau support conducteur à base de carbone contenu dans un réservoir à parois poreuses appartenant à une anode volumique,
en fin d'électrolyse, on dédope les motifs polymères ainsi obtenus dans la même boucle réactionnelle,
après dédopage des motifs polymères, on retire les moyens d'amenée du courant du réservoir,
puis on obture ledit réservoir qui contient le matériau support enrobés des motifs polymères, pour obtenir ainsi ladite cartouche filtrante.

19. Filtre d'épuration et/ou de récupération d'ions métalliques, plus particulièrement d'ions métalliques tels que l'argent, contenus dans des solutions amenées à son contact,
caractérisé en ce qu'il comprend au moins un réservoir à parois poreuses contenant un matériau à base de carbone enrobé de résine polymère selon l'une quelconque des revendications 9 à 11.
